(19) Europäisches Patentamt
European Patent Office
Office européen des brevets

(11) **EP 3 544 641 B1**

(12) **EUROPEAN PATENT SPECIFICATION**

(45) Date of publication and mention
of the grant of the patent:
**08.12.2021 Bulletin 2021/49**

(51) Int Cl.:
***C01B 25/32*** *(2006.01)*     ***C01B 25/45*** *(2006.01)*
***A61L 27/12*** *(2006.01)*

(21) Application number: **17864746.7**

(22) Date of filing: **29.10.2017**

(86) International application number:
**PCT/IB2017/056708**

(87) International publication number:
**WO 2018/078593 (03.05.2018 Gazette 2018/18)**

(54) **BIOMIMETIC APATITE NANOPOWDER COMPOSITION**

BIOMIMETISCHE APATITNANOPULVERZUSAMMENSETZUNG

COMPOSITION DE NANOPOUDRE D'APATITE BIOMIMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.10.2016 IR 1395300946**

(43) Date of publication of application:
**02.10.2019 Bulletin 2019/40**

(73) Proprietor: **Bakhshi, Farhad**
**Tehran (IR)**

(72) Inventors:
• **ABOUEI MEHRIZI, Ali**
**Tehran (IR)**
• **BASIRI, Hamideh**
**Tehran (IR)**
• **BAKHSHI, Farhad**
**Tehran (IR)**

(74) Representative: **Yavuzcan, Alev**
**Sumer Ofis**
**Patent and Trademark Agency Ltd. Co.**
**Ayaspasa Cami Sokak No.2/1**
**Gumussuyu Taksim**
**34437 Istanbul (TR)**

(56) References cited:
CN-A- 101 716 370      CN-A- 101 837 147
CN-A- 102 040 207      CN-A- 102 616 762
CN-A- 103 073 279      CN-A- 104 909 345
US-A1- 2012 165 577

• DAVID SHEPHERD ET AL: "Production of zinc substituted hydroxyapatite using various precipitation routes", BIOMEDICAL MATERIALS, vol. 8, no. 2, 23 January 2013 (2013-01-23), page 025003, XP055706731, GB ISSN: 1748-6041, DOI: 10.1088/1748-6041/8/2/025003
• ROBERT J. FRIEDERICHS ET AL: "Synthesis, characterization and modelling of zinc and silicate co-substituted hydroxyapatite", JOURNAL OF THE ROYAL SOCIETY. INTERFACE, vol. 12, no. 108, 1 July 2015 (2015-07-01), page 20150190, XP055706550, GB ISSN: 1742-5689, DOI: 10.1098/rsif.2015.0190
• FADEEV L I SHVORNEVA S M BARINOV V P ORLOVSKII I V: "SYNTHESIS AND STRUCTURE OF MAGNESIUM-SUBSTITUTED HYDROXYAPATITE", INORGANIC MATERIALS, PLENUM PUBLISHING CO., NEW YORK, NY, US, vol. 39, 1 January 2003 (2003-01-01), pages 947-950, XP001248852, ISSN: 0020-1685, DOI: 10.1023/A:1025509305805
• SPRIO ET AL: "Physico-chemical properties and solubility behaviour of multi-substituted hydroxyapatite powders containing silicon", MATERIALS SCIENCE AND ENGINEERING C, ELSEVIER SCIENCE S.A, CH, vol. 28, no. 1, 21 December 2007 (2007-12-21), pages 179-187, XP022398445, ISSN: 0928-4931, DOI: 10.1016/J.MSEC.2006.11.009
• ZOLTAN ZYMAN ET AL: "Sodium-carbonate co-substituted hydroxyapatite ceramics", PROCESSING AND APPLICATION OF CERAMICS, vol. 7, no. 4, 1 January 2013 (2013-01-01), pages 153-157, XP055706771, ISSN: 1820-6131, DOI: 10.2298/PAC1304153Z

- **CAIBAO XUE ET AL: "Hydrothermal Synthesis and Biocompatibility Study of Highly Crystalline Carbonated Hydroxyapatite Nanorods", NANOSCALE RESEARCH LETTERS, vol. 10, no. 1, 7 August 2015 (2015-08-07) , XP055706774, US ISSN: 1931-7573, DOI: 10.1186/s11671-015-1018-9**

**Description**

**TECHNICAL FIELD**

The present disclosure generally relates to biomimetic apatite nanopowder compositions and methods for synthesizing the biomimetic apatite nanopowder compositions.

**BACKGROUND ART**

Hydroxyapatite (HA) is widely used as a preventive agent and has the potential in remineralization of initial caries lesions. HA is one of the most effective calcium phosphate compounds for dental applications and fabrication of implants and bone augmentations due to its favorable characteristics such as biocompatibility and bioactivity. The performance of HA is significantly influenced by its particle size. Nano-sized HA is very similar to the apatite crystals of enamel in terms of morphology, crystal structure and chemical composition which make it a suitable material to rebuild enamel by improving the calcium and phosphate ions ability to promote remineralization process.

Bioactivity of synthetic hydroxyapatite can be enhanced by inclusion of intentionally added ionic substitutions, which create chemical compositions with more similarity to natural enamel, dentin or bone minerals. For example, zinc (Zn) is an important trace element that exists in the enamel of human teeth and bone as a substituent ion. It is one of the main substituent of calcium in apatite structure and its incorporation would change the crystallinity and particle sizes of the apatite crystals. Moreover, its presence in apatite structure can mimic the enamel compound. Regarding its advantages, Zn is extensively used as an antibacterial agent and formulated to oral hygiene products.

As another example, one of the elements associated with biological apatite is magnesium. Mg incorporation into HA stimulates osteoblast proliferation. Mg acts similar to a growth factor during the early stages of osteogenesis and promotes bone formation. The incorporation of Mg ions within the HA structure is essential for developing artificial bone substitutes.

Shepherd et al. (Biomedical Materials 8.2 (2013): Page 025003) have disclosed methods for synthesizing zinc substituted hydroxyapatite via two different precipitation routes. Their results showed that to produce stoichiometric hydroxyapatite using a reaction between calcium nitrate, zinc nitrate and ammonium phosphate, considerable amounts of ammonia are required to maintain a sufficiently high pH.

Friederichs et al. (Journal of The Royal Society Interface 12.108 (2015): 20150190) have disclosed a new ionic co-substitution in hydroxyapatite. Specifically, they have disclosed that complex co-substituted calcium phosphates may be utilized as synthetic bone mineral substitutes. Fadeev et al. (Inorganic Materials 39.9 (2003): 947-950) have synthesized magnesium-substituted calcium phosphates with the general formula $Ca_{10-x}Mg_x (PO_4)_6(OH)_2$ (x & 1). In their studies they have found that the magnesium ion may destabilize the structure of hydroxyapatite.

Spiro et al. (Materials Science and Engineering: C 28.1 (2008): 179-187) have synthesized hydroxyapatite powders characterized by ionic substitutions both in anionic and cationic sites in aqueous medium. Zyman et al. (Processing and Application of Ceramics 7.4 (2013): 153-157) have prepared sodium-carbonate co-substituted hydroxyapatite powders with a sodium content of 0.25 to 1.5 wt.% by a precipitation-solid state reaction route. Chen et al. (Nanoscale research letters 10.1 (2015): 1-6) have utilized a hydrothermal method to synthesize Highly crystalline carbonated hydroxyapatite (CHA) nanorods with different carbonate contents.

There is a need in the art for biomimetic hydroxyapatite compositions that can successfully mimic the natural HA composition of enamel, dentin, or bone. These biomimetic hydroxyapatite compositions may function as effective regenerating agents for enamel, dentin and bone, since they share physiochemical properties with the natural HA component of the enamel, dentin, or bone.

**SUMMARY OF THE DISCLOSURE**

This summary is intended to provide an overview of the subject matter of this patent, and is not intended to identify essential elements or key elements of the subject matter, nor is it intended to be used to determine the scope of the claimed implementations. The proper scope of this patent may be ascertained from the claims set forth below in view of the detailed description below and the drawings.

In one general aspect, the present disclosure is directed to a biomimetic apatite nanopowder composition that may have a general formula of :

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}$$

According to some implementations, in the above general formula, r may have a value between 8 and 10; m may have a value between 0 and 1.2; n may have a value between 0 and 2; z may have a value between 0 and 0.5; p may have a value between 4 and 6 such that $p - \dfrac{2}{3}a - s$ has a value between 3.54 and 6; a may have a value between 0 and 2; b may have a value between 0 and 1; s may have a value between 0 and 0.5. a and b are selected such that a+b may have a value between 0 and 2.

According to one implementation, the biomimetic apatite nanopowder composition may have a Ca to P ratio between 1.52:1 and 1.85:1. According to another implementation, the hydroxyapatite particles may include rod-shaped particles with a diameter between 3 and 30 nm, a length between 20 and 130 nm, and a length to diameter ratio between 4:1 and 15:1.

According to some implementations, in the above general formula, r may have a value between 8 and 10; m may have a value between 0.083 and 0.25; n may have a value 0.17 and 0.35; z may have a value between 0.03 and 0.05; p may have a value between 4 and 6 such that $p - \dfrac{2}{3}a - s$ has a value between 5.66 and 5.93; a may have a value between 0.105 and 0.52; b may have a value between 0.17 and 0.48; and s may have a value between 0 and 0.5. a and b are selected such that a+b may have a value between 0.58 and 0.68.

According to some implementations, in the above general formula, r may have a value between 8 and 10; m may have a value between 0.4 and 0.55; n may have a value 0.044 and 0.26; z may have a value between 0.015 and 0.03; p may have a value between 4 and 6 such that $p - \dfrac{2}{3}a - s$ has a value between 5.47 and 6; a may have a value between 0 and 0.8; b may have a value between 0.29 and 0.9; and s may have a value between 0 and 0.5. a and b are selected such that a+b may have a value between 0.9 and 1.09.

According to some implementations, in the above general formula, r may have a value between 8 and 10; m may have a value between 0.2 and 0.34; n may have a value 0.3 and 0.44; z may have a value between 0.0006 and 0.006; p may have a value between 4 and 6 such that $p - \dfrac{2}{3}a - s$ has a value between 5.17 and 5.54; a may have a value between 0.69 and 1.24; b may have a value between 0 and 0.4; and s may have a value between 0 and 0.018. a and b are selected such that a+b may have a value between 1.09 and 1.24.

## BRIEF DESCRIPTION OF THE DRAWINGS

The drawing figures depict one or more implementations in accord with the present teachings, by way of example only, not by way of limitation. In the figures, like reference numerals refer to the same or similar elements.

FIG. 1 shows X-ray diffraction (XRD) patterns of HA and Zn-HA samples, according to one or more exemplary embodiment of the present disclosure.

FIG. 2 shows Fourier-transform infrared (FTIR) spectrum of the synthesized HA and FTIR spectrum of Zn-HA nanopowders, according to one or more exemplary embodiment of the present disclosure.

FIG. 3A is a scanning electron microscope (SEM) image of the synthesized HA nanopowder, according to one exemplary embodiment of the present disclosure.

FIG. 3B is a SEM image of the synthesized Zn-HA nanopowder, according to one exemplary embodiment of the present disclosure.

FIG. 4 depicts percentage of surface microhardness recovery of enamel samples at various time periods throughout the pH-cycling procedure, according to one exemplary embodiment of the present disclosure.

FIG. 5A is a field emission scanning electron microscope (FE-SEM) micrograph of a sample of sound enamel, according to one exemplary embodiment of the present disclosure.

FIG. 5B is a FE-SEM micrograph of an enamel sample after 72 hours of demineralization, according to one exemplary embodiment of the present disclosure.

FIG. 5C is a FE-SEM micrograph of a HA layer formed on an initial enamel lesion, according to one exemplary embodiment of the present disclosure.

FIG. 5D is a FE-SEM micrograph of a Zn-HA layer formed on an initial enamel lesion, according to one exemplary embodiment of the present disclosure.

FIG. 5E is a FE-SEM micrograph of an initial enamel lesion treated by DDW, according to one exemplary embodiment of the present disclosure.

FIG. 6 shows scanning electron microscopic-energy dispersive X-ray spectroscopy (SEM-EDX) results for the surface of an enamel sample that was treated with Zn-HA.

FIG. 7 shows FTIR spectrum of the synthesized Mg-Na-CHA nanopowder, according to one exemplary embodiment of the present disclosure.

**FIG. 8** shows XRD pattern of the synthesized Mg-Na-CHA nanopowder, according to one exemplary embodiment of the present disclosure.

**FIG. 9** shows FTIR spectrum of the synthesized Zn-Na-CHA nanopowder, according to one exemplary embodiment of the present disclosure.

**FIG. 10** shows XRD pattern of the synthesized Zn-Na-CHA nanopowder, according to one exemplary embodiment of the present disclosure.

## DESCRIPTION OF EMBODIMENTS

In the following detailed description, numerous specific details are set forth by way of examples in order to provide a thorough understanding of the relevant teachings. However, it should be apparent that the present teachings may be practiced without such details. In other instances, well known methods, procedures, components, and/or circuitry have been described at a relatively high-level, without detail, in order to avoid unnecessarily obscuring aspects of the present teachings.

Disclosed compositions are directed to biomimetic apatite nanopowder compositions that may be synthesized by intentionally adding or otherwise incorporating ionic substitutes into a structure of a synthetic hydroxyapatite (HA). In some implementations of the disclosed compositions, ionic substitutes, both anionic and cationic, are included in the structure of synthetic HA in order to mimic the HA component of enamel, dentin, and/or bone. The biomimetic apatite nanopowder compositions of the present disclosure include cationic and anionic substitutes with predetermined amounts that are close to the amounts of the cationic and anionic substitutes in natural HA in enamel, dentin, and/or bone. The predetermined amounts are selected such that the biomimetic apatite nanopowder compositions of the present disclosure share the physiochemical properties of natural HA in enamel, dentin, and/or bone.

In an aspect, the biomimetic apatite nanopowder composition may include cationic substitutes such as calcium, zinc, magnesium, and sodium ions; and anionic substitutes such as silicate, carbonate and phosphate ions with a general Formula (1):

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s} \qquad \text{Formula (1)}$$

According to some implementations, in the Formula (1), r may have a value between 8 and 10; m may have a value between 0 and 1.2; n may have a value between 0 and 2; z may have a value between 0 and 0.5; p may have a value between 4 and 6 such that $p - \frac{2}{3}a - s$ has a value between 3.54 and 6; a may have a value between 0 and 2; b may have a value between 0 and 1; and s may have a value between 0 and 0.5. a and b are selected such that a+b may have a value between 0 and 2. In the Formula (1), ratio of Ca to P may be between 1.52:1 and 1.85:1.

According to one exemplary embodiment, the biomimetic apatite nanopowder composition of the present disclosure may be utilized as a remineralization agent for remineralizing caries lesions in an enamel tissue. In order to use the biomimetic apatite nanopowder composition of the present disclosure for remineralizing caries lesions in an enamel tissue, the biomimetic apatite nanopowder composition may mimic the natural HA in enamel, and therefore in the Formula (1), r may have a value between 8 and 10; m may have a value between 0.083 and 0.25; n may have a value 0.17 and 0.35; z may have a value between 0.03 and 0.05; p may have a value between 4 and 6 such that $p - \frac{2}{3}a - s$ has a value between 5.66 and 5.93; a may have a value between 0.105 and 0.52; b may have a value between 0.17 and 0.48; and s may have a value between 0 and 0.5. a and b are selected such that a+b may have a value between 0.58 and 0.68.

According to one exemplary embodiment, the biomimetic apatite nanopowder composition of the present disclosure may be utilized as a remineralization agent for remineralizing caries lesions in a dentin tissue. In order to use the biomimetic apatite nanopowder composition of the present disclosure for remineralizing caries lesions in a dentin tissue, the biomimetic apatite nanopowder composition may mimic the natural HA in dentin, and therefore in the Formula (1), r may have a value between 8 and 10; m may have a value between 0.4 and 0.55; n may have a value 0.044 and 0.26; z may have a value between 0.015 and 0.03; p may have a value between 4 and 6 such that $p - \frac{2}{3}a - s$ has a value between 5.47 and 6; a may have a value between 0 and 0.8; b may have a value between 0.29 and 0.9; and s may have a value between 0 and 0.5. a and b are selected such that a+b may have a value between 0.9 and 1.09.

According to one exemplary embodiment, the biomimetic apatite nanopowder composition of the present disclosure may be utilized as a remineralization agent for remineralizing lesions in a bone tissue. In order to use the biomimetic apatite nanopowder composition of the present disclosure for remineralizing lesions in a bone tissue, the biomimetic apatite nanopowder composition may mimic the natural HA in bone, and therefore in the Formula (1), r may have a value between 8 and 10; m may have a value between 0.2 and 0.34; n may have a value 0.3 and 0.44; z may have a value between 0.0006 and 0.006; p may have a value between 4 and 6 such that $p - \frac{2}{3}a - s$ has a value between 5.17 and 5.54; a may have a value between 0.69 and 1.24; b may have a value between 0 and 0.4; and s may have a value between 0 and 0.018. a and b are selected such that a+b may have a value between 1.09 and 1.24.

In another aspect, the present disclosure is directed to a method for synthesizing the biomimetic apatite nanopowder composition of the present disclosure. According to some implementations, the method for synthesizing the biomimetic apatite compositions of the present disclosure may include: preparing a first solution of cationic compounds; preparing a second solution of anionic compounds; adding the second solution to the first solution in a dropwise manner while being agitated; aging the mixture of the first and the second solution; separating the precipitated salt from the aged mixture; and optionally drying and grinding the precipitated salt in order to obtain the biomimetic apatite nanopowder.

According to one implementation, with respect to the step of preparing the first solution of cationic compounds, the cationic compounds may include a zinc (Zn) source, a Ca source, a Na source, and an Mg source. According to some implementations, the Zn source may be selected from the group consisting of a zinc acetate such as zinc acetate dehydrate, a zinc nitrate such as zinc nitrate hexahydrate, a zinc citrate such as zinc citrate dehydrate, zinc fluoride, zinc chloride, zinc carbonate, and combinations thereof. According to some implementations, the Mg source may be selected from the group consisting of a magnesium chloride such as magnesium chloride anhydrous or magnesium chloride hexahydrate, magnesium fluoride, a magnesium nitrate such as magnesium nitrate hexahydrate, magnesium oxide, magnesium hydroxide, magnesium carbonate, magnesium acetate, and combinations thereof. According to other implementations, the Na source may be selected from the group consisting of a sodium carbonate such as sodium carbonate anhydrous, sodium nitrate, sodium bicarbonate, sodium fluoride, sodium hydroxide, sodium chloride, and combinations thereof. According to one or more implementations, the Ca source may be selected from the group consisting of calcium fluoride, calcium chloride, calcium nitrate, calcium carbonate, calcium hydroxide, calcium phosphate, calcium acetate, and combinations thereof.

According to one implementation, with respect to the step of preparing the second solution of anionic compounds, the anionic compounds may include a carbonate source, a phosphate source. It should be mentioned that silicate, which is an anionic compound must be added to the first cationic solution not the second anionic solution. According to some implementations, the phosphate source may be selected from the group consisting of disodium hydrogen phosphate, sodium dihydrogen phosphate, orthophosphoric acid, dipotassium hydrogen phosphate, potassium dihydrogen phosphate, di-ammonium hydrogen phosphate, and combinations thereof. According to other implementations, the carbonate source may be selected from the group consisting of a sodium carbonate such as sodium carbonate anhydrous, sodium bicarbonate, potassium carbonate, and combinations thereof. According to one or more implementations, the silicate source may be selected from the group consisting of sodium silicate, tetraethyl orthosilicate, silicon acetate, and combinations thereof.

According to one implementation, with respect to the step of adding the second solution to the first solution in a dropwise manner while being agitated, the addition may be carried out at a temperature between room temperature and 38 °C and during the agitation the pH may be kept between 8.5 and 11. In order to adjust the pH level, alkaline or acidic solutions may be added to the mixture. According to one exemplary embodiment, the first solution may be added dropwise to the second solution with a rate of at least 0.12 mlmin$^{-1}$ and at most 15 mlmin$^{-1}$.

According to another implementation, with respect to the step of aging the mixture of the first and the second solution, the mixture may be aged for 96 hours. According to some implementations, with respect to the step of separating the precipitated salt from the aged mixture, separation may be carried out by centrifugation, lyophilizing, spray drying or vacuum drying. In case of centrifugation the final product is obtained as a paste which can be directly used or the paste can be dried to obtain a powder.

## Example 1: Synthesis of Nanocrystalline Hydroxyapatite (HA) Nanopowder and Nanocrystalline Zn-doped Hydroxyapatite (Zn-HA) Nanopowder

In this exemplary embodiment, a nanocrystalline Zn-HA nanopowder is synthesized by the following method. A 0.24 M aqueous solution of diammonium hydrogen phosphate ($[(NH_4)_2HPO_4$) was added dropwise to 0.28 M solution of calcium nitrate tetrahydrate ($Ca(NO_3)_2.4H_2O$) with continuous stirring. Sodium hydroxide (NaOH) aqueous solution was gradually added to adjust the pH of the solution to 11. At the end of the reaction, the suspension was aged for 24 h at room temperature while being stirred with a magnetic stirrer. Then the resultant HA suspension was centrifuged and washed with hot distilled water, a process that was repeated for at least three times. The resultant HA suspension was then heated for 24 h in an oven at a temperature of 70 °C to obtain the nanocrystalline HA nanopowder.

In this exemplary embodiment, a nanocrystalline Zn-HA nanopowder is also synthesized by the following method. A 0.24 M aqueous solution of diammonium hydrogen phosphate ($[(NH_4)_2HPO_4$) was added dropwise to 0.28 M solution of calcium nitrate tetrahydrate ($Ca(NO_3)_2.4H_2O$) and zinc nitrate hexahydrate ($Zn(NO_3)_2.6H_2O$) with continuous stirring. Molar ratio of Zn to Ca was 0.052:1. Sodium hydroxide (NaOH) aqueous solution was gradually added to adjust the pH of the solution to 11. At the end of the reaction, the suspension was aged for 24 h at room temperature while being stirred with a magnetic stirrer. Then the resultant Zn-HA suspension was centrifuged and washed with hot distilled water, a process that was repeated for at least three times. The resultant Zn-HA suspension was then heated for 24 h in an oven at a temperature of 70 °C to obtain the nanocrystalline Zn-HA nanopowder.

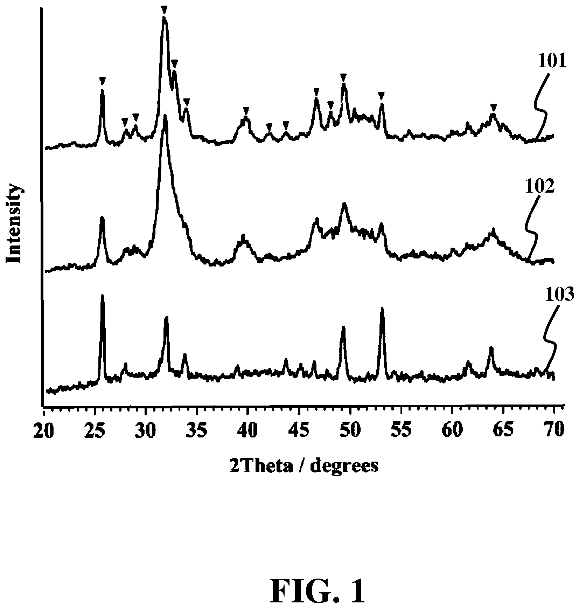

**FIG.** 1 shows X-ray diffraction (XRD) patterns of HA and Zn-HA samples (referred to by reference numbers **101** and **102,** respectively) synthesized as described in Example 1, and a natural enamel sample (referred to by reference numbers **103).** The diffraction pattern **101** of the HA sample was assigned to the standard pattern of HA (JCPDS No. 09-0432). The diffraction peaks at 2theta values of 31.76, 32.92, and 25.88 degrees are consistent with the (211), (300), and (002) Bragg reflections of HA with a hexagonal crystalline structure, respectively. Sharp peaks of the diffraction pattern **101** of the HA sample, indicates that HA nanopowder is crystallized. No impurities like tricalcium phosphate or calcium oxide are detected in the HA sample. Referring to **FIG. 1,** with respect to the diffraction pattern **102** of the Zn-HA sample, all peaks were assigned to the standard pattern (JCPDS No. 09-0432). No peaks corresponding to the second phases like calcium hydroxide or zinc phosphate were detected.

Utilizing the XRD data, lattice parameters (*a* and c) of the unit cells of a hexagonal crystalline structures can be calculated by Equation (1) below:

$$\frac{1}{d^2} = \frac{4(h^2 + hk + k^2)}{3a^2} + \frac{l^2}{c^2} \qquad \text{Equation (1)}$$

The calculated lattice parameters of HA and Zn-HA nanopowders and the natural enamel sample are presented in **Table 1.** Referring to the data calculated and set forth in **Table 1,** incorporation of Zn species into the crystal structure of HA results in a reduction of lattice parameters *a* and *c* of the Zn-HA nanopowder in comparison with the HA and enamel samples. Since the atomic radius of Zn, which is 0.074 nm is smaller than that of Ca, which is 0.099 nm, the substitution of Ca ions in the HA structure with Zn, results in a reduction of interplanar spacing of the crystal structure and consequently leads to a slight shift in the peak positions in Zn-HA diffraction pattern **102** as shown in **FIG. 1.**

**Table 1:** Lattice parameters of synthesized HA and Zn-HA nanopowders and the natural enamel sample.

| Sample | *a* (Å) | *c* (Å) |
|---|---|---|
| HA | 9.4180 | 6.8828 |
| Zn-HA | 9.4167 | 6.8764 |
| Natural Enamel | 9.4400 | 6.8814 |

The XRD data were also used to calculate the degree of crystallinity of the synthesized HA and Zn-HA nanopowders and the natural enamel sample, using Equation (2) below:

$$X_c = 1 - (V_{112/300}/I_{300}) \qquad \text{Equation (2)}$$

In Equation (2), $I_{300}$ is the intensity of (300) reflection and $V_{112/300}$ is the intensity of the hollow between (112) and (300) reflections. The results of calculations are presented in **Table 2.** Referring to data reported in **Table 2,** it can be understood that incorporation of Zn ions in HA crystal structure results in broadening of the diffraction pattern peaks and consequently deceases the degree of crystallinity of the Zn-Ha nanopowder. Peak broadening indicates a low degree of crystallinity about 46% and 28% for the synthesized HA and Zn-HA nanopowders, respectively. While the degree of crystallinity for natural enamel is about 67.78%.

In addition, Scherrer's equation was used to determine the crystallite size of the synthesized HA and Zn-HA nanopowders. The Scherrer's equation is presented as Equation (3) below:

$$D_{hkl} = K\lambda / \beta_{1/2}Cos\theta \qquad \text{Equation (3)}$$

In Equation (3), $\lambda$ is the wavelength (Cu K$\alpha$), $\beta_{1/2}$ is the full width at half-maximum of the selected peak in the XRD patterns (shown in **FIG. 1)** and θ is the diffraction angle (referred to as "theta" in **FIG. 1).** The (002), (310), and (222) peaks were used for the calculations. Results of crystallite size calculations are presented in **Table 3.** The average crystallite size decreases in Zn-HA nanopowder, which indicates that Zn substitution, inhibits crystal growth of HA.

**Table 2:** Degree of crystallinity of synthesized HA and Zn-HA nanopowders and the natural enamel sample.

| Samples | 2θ | Variable | Intensity(counts) | Crystallinity (%) |
|---|---|---|---|---|
| HA | 32.78 | V112/300 | 56 | 46.15 |
| | 32.92 | 1300 | 104 | |
| Zn-HA | 32.78 | V112/300 | 85 | 28.57 |
| | 32.9 | 1300 | 119 | |
| Enamel | 32.74 | V112/300 | 29 | 67.78 |
| | 32.92 | 1300 | 90 | |

**Table 3:** Crystallite size of the synthesized HA and Zn-HA nanopowders and the natural enamel sample.

| Sample | Crystallite Size (nm) | | | |
|---|---|---|---|---|
| | 002 | 310 | 222 | Average |
| HA | 31.55 | 43.56 | 47.29 | 40.80 |
| ZnHA | 22.03 | 32.49 | 31.007 | 28.509 |
| Enamel | 34.52 | 34.91 | 26.19 | 31.87 |

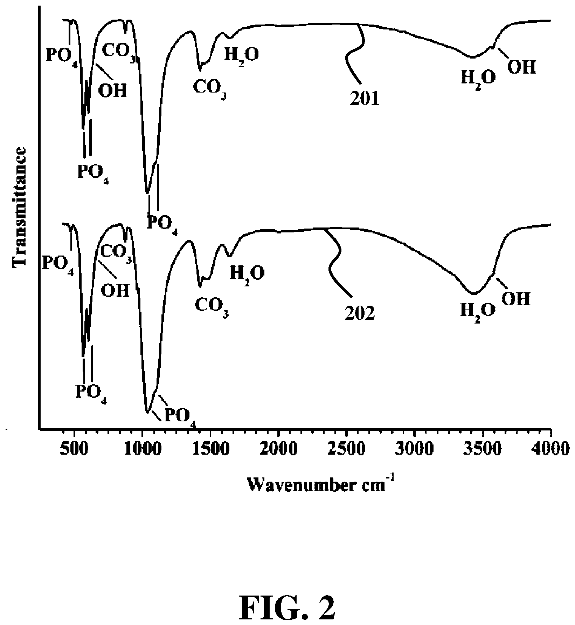

**FIG. 2** shows Fourier-transform infrared (FTIR) spectrum **201** of the synthesized HA and FTIR spectrum **202** of Zn-HA nanopowders. The FTIR spectra **201, 202** of the synthesized HA and Zn-HA nanopowders consist of several peaks at 472, 564, 603, 962, and 1032 cm$^{-1}$ which are assigned to the $PO_4^{3-}$ bands. Moreover, another peak associated with OH$^-$ functional group at 3569 cm$^{-1}$ appeared in the FTIR spectrum **201** of HA nanopowder. In addition, several broad peaks in the range of 3000-3500 cm$^{-1}$ and a sharp peak at 1640 cm$^{-1}$ could be attributed to the water which is adsorbed due to the high specific area of the nanopowders. Referring to **FIG. 2,** the peaks associated with $CO_3^{2-}$ functional groups appeared at 875, 1422 and 1451 cm$^{-1}$, which can be substituted to both $PO_4^{3-}$ and OH$^-$ ions.

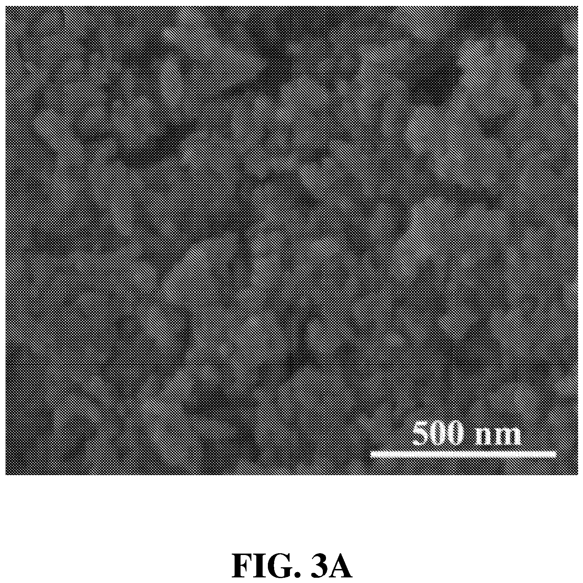

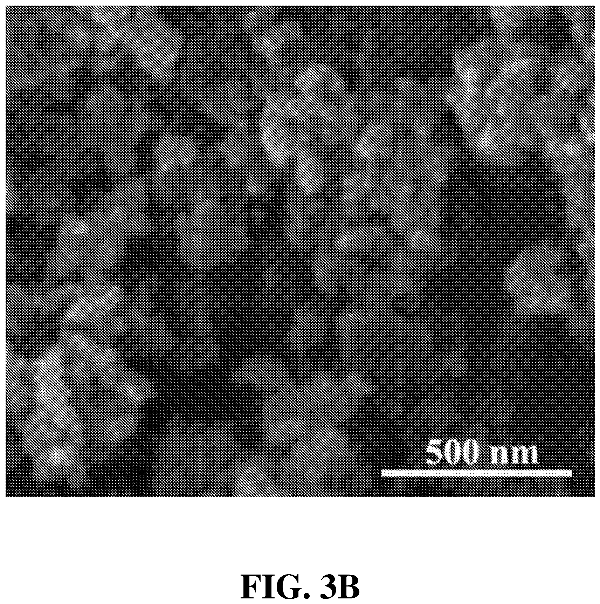

**FIG. 3A** is a scanning electron microscope (SEM) image of the synthesized HA nanopowder, according to one exemplary embodiment of the present disclosure and **FIG. 3B** is a SEM image of the synthesized Zn-HA nanopowder, according to one exemplary embodiment of the present disclosure. Referring to **FIGs. 3A** and **3B,** the particle size of the synthesized HA nanopowder was measured to be between 37 and 70 nanometers, while the particle size of the synthesized Zn-HA nanopowder was measured to be in a range of 25 to 50 nanometers. The particle size of the synthesized Zn-HA nanopowder was smaller than that of the synthesized HA nanopowder. Moreover, with reference to **FIGs. 3A** and **3B,** agglomerations of small spherical particles were observed in nanometric scale for both the synthesized HA nanopowder and the synthesized Zn-HA nanopowder.

In an example, 40 human molars extracted teeth without any caries, enamel fractures and cracks (hereinafter "enamel samples") were collected and used for in vitro characterizations. All enamel samples were thoroughly cleaned of any debris and stored in a 0.5 % of Chloramine-T solution for less than 7 days and finally were kept in saline solution at a temperature of approximately 4 °C in a refrigerator before use. Buccal surfaces of enamel samples were separated by a water-cooled diamond saw microtome (Mecatome A 201T, Presi, France) and each slab was embedded in repair cold cure dental acrylic resin. The superficial enamel surfaces were ground down on wet abrasive SiC papers (2000, 2500, 3000 grit) to provide a flat surface. A 3 mm × 3 mm area was created on each enamel sample's surface with acid-resistant nail polish (Coloroma nail varnish, Maybelline).

In order to simulate the dynamic process that occurs in the oral cavity, all of the enamel samples were initially treated to create an artificial lesion thereon and then all of the enamel samples were subjected to a pH cycling. To create the initial artificial lesions, each enamel slab was separately immersed in 8 mL of a demineralization solution (2.0 mmolL$^{-1}$ calcium (calcium nitrate), 2.0 mmolL$^{-1}$ phosphate (potassium dihydrogen phosphate), 75 mmolL$^{-1}$ acetic acid, pH 4.4) for 72 h at 37 °C. In addition, a pH-cycling model was used to simulate the dynamic demineralization-remineralization process that occurs in the oral cavity.

The synthesized HA, Zn-HA nanopowders, and deionized water were used as remineralization agents. **Table**

**4** presents the experimental design in the treatment regimen. Enamel samples were divided into three groups based on their remineralization agents. The remineralization agents in each group were prepared by adding the respective remineralization nanopowder to the distilled deionized water (DDW). According to the selected pH cycling model, each of the enamel samples was initially treated with the respective remineralizing agents for three minutes, washed with DDW, and then dried and after that each of the enamel samples was immersed in 20 mL of the demineralizing solution for a period of 6 h. Then, after being washed with DDW and dried, the enamel samples were treated with the respective remineralizing agents for another three minutes. Finally, after being washed with DDW and dried, the enamel samples were immersed in the remineralizing solution (1.5 mmolL$^{-1}$ calcium, 0.9 mmolL$^{-1}$ phosphate, 130 mmolL$^{-1}$ potassium chloride and 20.0 mmolL$^{-1}$ sodium cacodylate, pH 7.0) for a period of 16 h. This procedure was repeated for 14 days while temperature was maintained at 37 °C in an incubator. Solutions were continuously stirred during experiments. Also demineralizing and artificial saliva solutions were freshly prepared every three days while treatment remineralizing solutions were prepared every day. Remineralization agents were dispersed ultrasonically.

**Table 4:** The pH-cycling experiment design in the treatment regimen.

| pH-Cycling process day | Time |
|---|---|
| Rinse with distilled water | |
| Treatment solutions | 3 minutes |
| Rinse with distilled water | |
| Demineralization Solution | 6 hours |
| Rinse with distilled water | |
| Treatment solutions | 3 minutes |
| Rinse with distilled water | |
| Remineralization solution | 16 hours |

The surface microhardness of baseline enamel samples (SMH1) prior to pH-cycling and after 3, 7, and 14 days of pH-cycling was measured by using Vickers microhardness test (Micro Vickers Hardness tester, SCTMC Co., Ltd, Shanghai, China). The microhardness values are the mean values of at least three measurements. The mean values of all measurements for different enamel samples were then compared and the percentage of surface microhardness recovery (%SMHR) was calculated by Equation (4) below, where n represents 3, 7, and 14 days of application and SMH is the initial microhardness of the enamel sample before demineralization and SMH1 is the initial microhardness of the enamel sample after 72 hours of demineralization:

$$(\%SMHR) = (SMHn\text{-}SMH1)/(SMH\text{-}SMH1)\times100 \qquad\qquad \text{Equation (4)}$$

Data analysis was performed using SPSS 20.0 software. SMH and percentage of surface microhardness recovery (%SMHR) among groups were analyzed using one-way ANOVA and followed by the Tukey's post hoc test. The significance level was set at 0.05.

Slurries containing 10% synthesized HA and Zn-HA nanopowders were used to evaluate the potential of Zn-subsituted HA as a remineralization agent under the pH cycling model. Surface microhardness of different enamel samples in each group was measured before and after pH cycling to evaluate the remineralization performance of the synthesized HA nanopowder. The results of microhardness evaluation for different enamel samples are reported in **Table 5.** As expected, before pH cycling all enamel samples dehardened significantly and surface enamel microhardness decreased. Since pH of the demineralization solution is lower than critical pH, calcium and phosphate leached out of the enamel samples and microhardness is reduced significantly, while microhardness of all groups has increased after pH cycling.

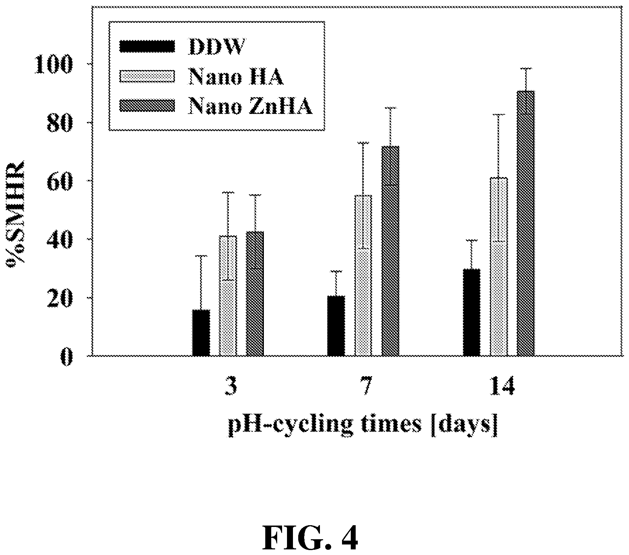

**FIG. 4** depicts percentage of surface microhardness recovery (%SMHR) of enamel samples at various time periods throughout the pH-cycling procedure. Referring to **FIG. 4,** in contrast to the negative control group, %SMHR of HA and Zn-HA groups were significantly improved.

Table 5: Surface microhardness analysis of enamel samples at various time periods throughout the pH-cycling procedure.

| Treatment Groups | SMH | | | | |
|---|---|---|---|---|---|
| | Baseline | before pH-cycling | pH-cycling | | |
| | | | 3 days | 7 days | 14 days |
| HA | $407.26 \pm 40.5^a$ | $277.65 \pm 40.7^a$ | $328.20 \pm 34.6^a$ | $354.69 \pm 21.0^{a,c}$ | $365.43 \pm 19.7^c$ |
| Zn-HA | $408.01 \pm 21.6^a$ | $282.90 \pm 56.3^a$ | $340.09 \pm 30.1^{a,b}$ | $376.69 \pm 22.4^a$ | $399.69 \pm 15.3$ |
| DDW | $407.38 \pm 41.4^a$ | $275.46 \pm 51.8^a$ | $296.99 \pm 45.2^{b,c}$ | $300.94 \pm 45.3^c$ | $312.10 \pm 43.7$ |

Same characters (a, b) are not significantly different within the same time period in different treatments at $p > 0.05$. Means followed by letter (c) shows no significant difference at different time periods for each treatment atp > 0.05.

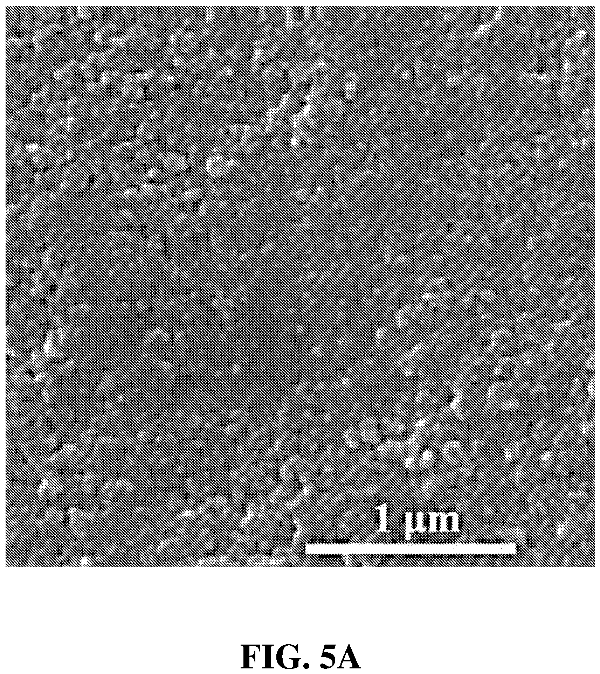

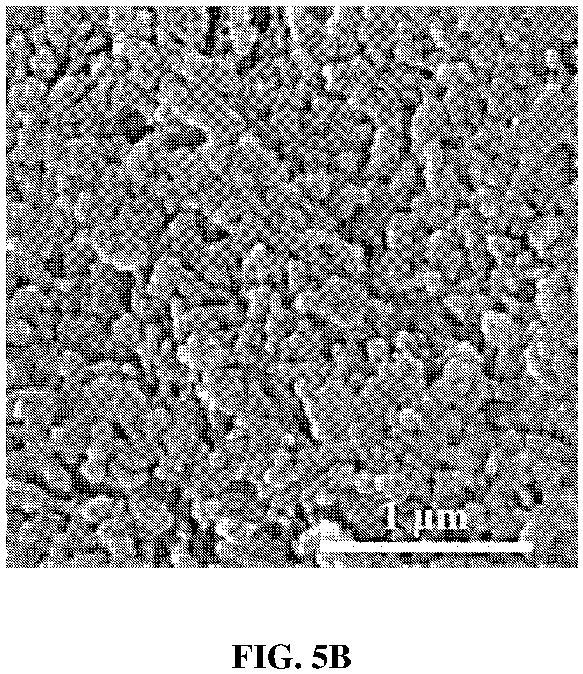

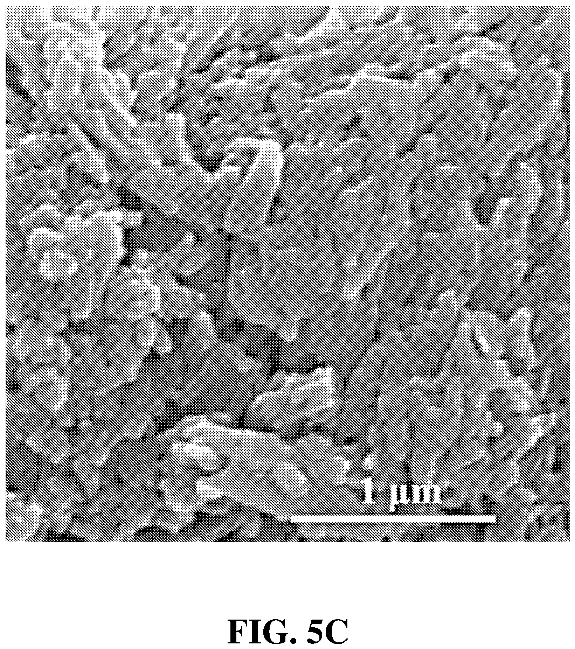

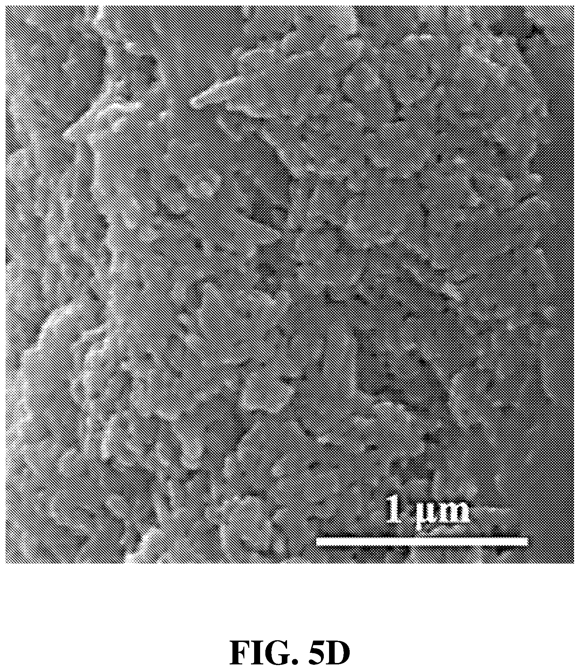

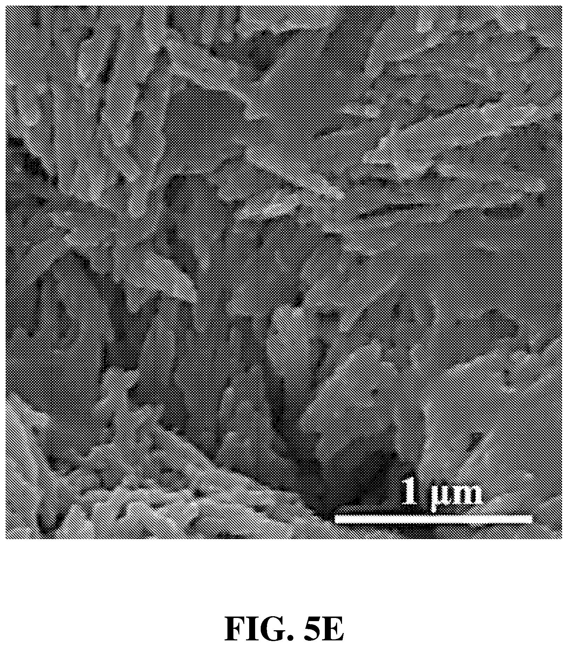

**FIG. 5A** is a field emission scanning electron microscope (FE-SEM) micrograph of a sample of sound enamel; **FIG. 5B** is a FE-SEM micrograph of a enamel sample after 72 hours of demineralization; **FIG. 5C** is a FE-SEM micrograph of a HA layer formed on an initial enamel lesion; **FIG. 5D** is a FE-SEM micrograph of a Zn-HA layer formed on an initial enamel lesion; and **FIG. 5E** is a FE-SEM micrograph of an initial enamel lesion treated by DDW. Referring to **FIG. 5B,** many defects and porosities are observable on the damaged surface of the enamel sample. With reference to **FIGs. 5C** and **5D,** after pH-cycling, it is observed that remineralized enamel is uniformly covered by a layer formed by the synthesized HA and Zn-HA nano nanopowders. However, with reference to **FIG. 5E,** in DDW group, the enamel sample still became demineralized and large porosities remain after treatment.

Referring to **FIG. 5B,** demineralization of the enamel surface below critical pH, increased the surface porosities. These defects could act as active sites for deposition of apatite particles during remineralization treatment. Referring to **FIGs. 5C** and **5D,** the improvement of remineralization process of the enamel sample, which was subjected to HA and Zn-HA agents could be attributed to strong adsorption of these biomimetic nanopowders. It is observed that the surface of the demineralized enamel sample is uniformly covered with new biomimetic HA and Zn-HA nanopowders. Morphology and composition are significant factors in remineralization process. Inorganic portions of the enamel structure consist of a biological apatite which is composed of impure calcium phosphate. Supersaturated levels of the calcium, phosphate, and zinc ions in close proximity to the tooth surface may enhance the formation of hydroxyapatite crystals in demineralized lesions and consequently reduce the enamel demineralization. The layers of HA or Zn-HA can act as a protective layer and increase the teeth hardness. Also, The layers of HA or Zn-HA may decrease the leaching of calcium and phosphate out of the enamel and prevent diffusion of acids into the deep layer of enamel surface and also inhibit the formation of cavitation infections.

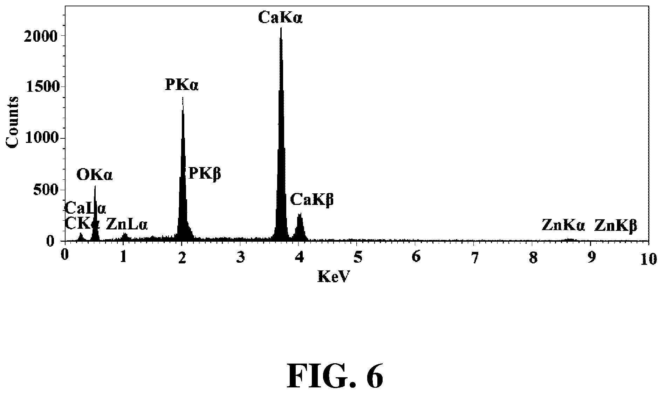

**FIG. 6** shows scanning electron microscopic-energy dispersive X-ray spectroscopy (SEM-EDX) results for the surface of an enamel sample that was treated with Zn-HA. Referring to **FIG. 6,** the SEM-EDX results clearly show the presence of Zn and C, as well as calcium, phosphorous, and oxygen in the structure. These results were consistent with XRD (shown in **FIG. 1)** and FTIR (shown in **FIG. 2)** results and shows that by treatment with Zn-HA, all of nanopowder ions contributed in remineralization process.

Referring to **Table 5,** teeth in Zn-HA group showed significantly higher microhardness recovery than the other two groups. It has been reported that the presence of zinc ions near a demineralized portion of the enamel, promotes the formation of zinc phosphate layer which is more acid resistant and could prevent further mineral loss. In a remineralization agent that includes an active Zn-HA nanopowder, dynamic demineralization-remineralization processes may occur in the following steps. First, adsorption of Zn-HA nanopowder on the active sites, form a new biomimetic layer on the enamel surfaces that are exposed to the remineralization agent; then, as the enamel is subjected to acid solution, the adsorbed layer dissolves and releases its constituent ions; after that, the dissolution of the adsorbed layer continues until a supersaturated remineralizing condition occurs near the enamel surface, which may result in the formation of zinc phosphate species through a co-precipitation or ion exchange process. The superior performance of the synthesized Zn-HA nanopowder could also be attributed to smaller particle size of the synthesized Zn-HA nanopowder, which may increase the adsorption characteristics and allows the particles to uniformly fill the defects and cover the enamel surface. Moreover, the presence of Zn in the HA structure can mimic the structure and chemical composition of the natural enamel and therefore improve the efficiency of HA.

In Example 1, HA and Zn-HA nanopowders were synthesized using a precipitation method. Physicochemical characterization revealed that Zn was completely incorporated to the HA structure and influenced the lattice parameters, crystallite size, and degree of crystallinity of the HA. It was revealed that both new biomimetic nanopowders can be effective in remineralization of initial caries lesions. Zn-HA shows higher remineralization ability compared to that of HA and can be effectively used as a remineralization agent in dental hygiene products such as toothpaste, mouthwashes, and oral health compounds.

**Example 2: Synthesis of Nanocrystalline Mg, Na-doped Carbonated Hydroxyapatite (Mg-Na-CHA) Nanopowder**

In this exemplary embodiment, a nanocrystalline Mg-Na-CHA nanopowder with a formula of $Ca_{9.55}Mg_{0.4}Na_{0.1}(CO_3)_{0.1}(PO_4)_{5.97}(OH)_{1.9}$ is synthesized by the following method. Approximately 88.47 g of $Ca(OH)_2$, 12.44 g of $Mg(NO_3)_2.6H_2O$ were dissolved in 1200 ml deionized distilled water (DDW) to obtain a cationic solution. Approximately 1.02 g of $NaHCO_3$ was dissolved in 40 ml DDW, and 82.57 g $H_3PO_4$ was dissolved in 560 ml DDW while being stirred. After that, prepared $NaHCO_3$ solution and $H_3PO_4$ solution were added in a dropwise manner into the cationic solution in a period of approximately 270 minutes in order to obtain a first suspension. The rate of addition of $NaHCO_3$ and $H_3PO_4$ were 0.15 and 2.2 ml/min, respectively. Then the first suspension was aged for 60 hr. After that, the first suspension was washed three times with DDW and centrifuged at 4000 rpm for approximately 20 minutes to obtain a paste. The paste was dried in an oven at a temperature of approximately 70°C, overnight to obtain a solid composition. The solid composition was then milled to obtain the Mg-Na-CHA nanopowder.

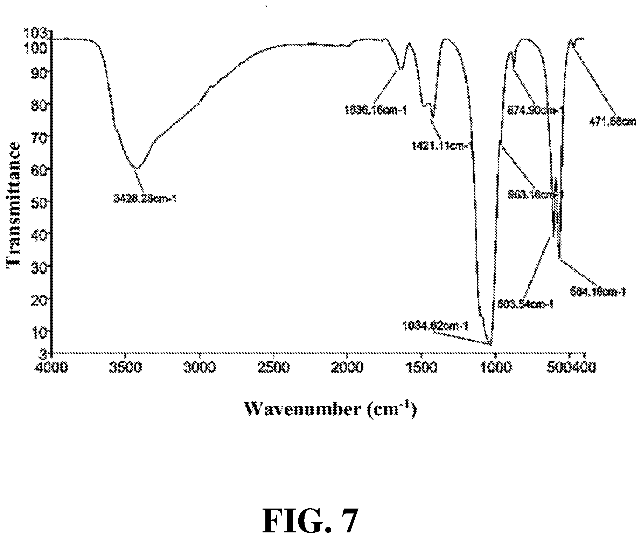

**FIG. 7** shows FTIR spectrum of the synthesized Mg-Na-CHA nanopowder. The FTIR spectrum of the synthesized Mg-Na-CHA nanopowder consist of several peaks at 471.68, 584.19, 603.54, 963.18, and 1034.62 cm$^{-1}$ which are assigned to the $PO_4^{3-}$ bands. Moreover, another peak associated with OH$^-$ functional group at 3569 cm$^{-1}$ appeared in the FTIR spectrum of the synthesized Mg-Na-CHA nanopowder. In addition, several broad peaks in the range of 3000-3500 cm$^{-1}$ and a sharp peak at 1636.16 cm$^{-1}$ could be attributed to the water which is adsorbed due to the high specific area of the synthesized Mg-Na-CHA nanopowder. Referring to **FIG. 7,** the peaks associated with $CO_3^{2-}$ functional groups appeared at 874.9, and 1421.11 cm$^{-1}$, which can be substituted to both $PO_4^{3-}$ and OH$^-$ ions.

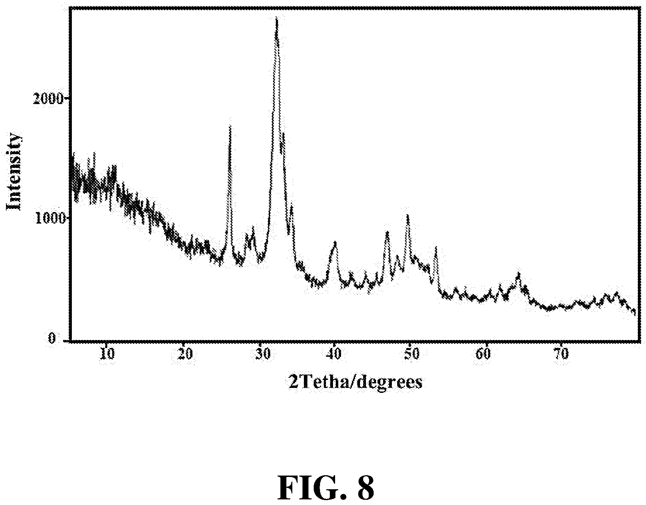

**FIG. 8** shows XRD pattern of the synthesized Mg-Na-CHA nanopowder, where all peaks were assigned to the standard pattern (JCPDS No. 09-0432). No peaks corresponding to the second phases like calcium hydroxide or zinc phosphate were detected. With reference to **FIG. 8,** the XRD data extracted from the XRD pattern of the synthesized Mg-Na-CHA nanopowder were used to calculate the lattice parameters (*a* and c) of the unit cells of the hexagonal crystalline structure of the synthesized Mg-Na-CHA nanopowder by Equation (1). The calculated average lattice parameters of the synthesized Mg-Na-CHA nanopowder are presented in **Table 6.**

**Table 6:** Lattice parameters of the synthesized Mg-Na-CHA nanopowder.

| 2θ | h | k | l | d$_{(Å) \text{ (from graph)}}$ | a (Å) | c (Å) |
|---|---|---|---|---|---|---|
| 18.7850 | 1 | 1 | 0 | 4.7212 | 9.4424 | 0 |
| 21.8190 | 2 | 0 | 0 | 4.0698 | 9.3988 | 0 |
| 25.8790 | 0 | 0 | 2 | 3.4294 | 0.0000 | 6.8588 |
| 28.9660 | 2 | 1 | 0 | 3.0785 | 9.4050 | 0 |
| 32.9020 | 3 | 0 | 0 | 2.7185 | 9.4172 | 0 |
| 39.8180 | 3 | 1 | 0 | 2.2608 | 9.4126 | 0 |
| 44.3690 | 4 | 0 | 0 | 2.0404 | 9.4241 | 0 |
| 48.6230 | 3 | 2 | 0 | 1.8711 | 9.4176 | 0 |
| 51.2830 | 4 | 1 | 0 | 1.7814 | 9.4263 | 0 |
| 53.1430 | 0 | 0 | 4 | 1.7203 | 0.0000 | 6.8812 |
| 59.9380 | 4 | 2 | 0 | 1.5420 | 9.4215 | 0 |
| 63.4430 | 5 | 1 | 0 | 1.4655 | 9.4216 | 0 |
| | | | | **Average** | 9.4187 | 6.8700 |

**Example 3: Synthesis of Nanocrystalline Zn, Na-doped Carbonated Hydroxyapatite (Zn-Na-CHA) Nanopowder**

In this exemplary embodiment, a nanocrystalline Zn-Na-CHA nanopowder with a formula of $Ca_{9.85}Zn_{0.052}Na_{0.2}(CO_3)_{0.14}(PO_4)_{5.94}(OH)_{1.9}$ is synthesized by the following method. Approximately 22.632 g of $Ca(OH)_2$, 0.344 g of $C_4H_6O_4Zn.2H_2O$, and 0.0723 g of NaOH were dissolved in 1000 ml DDW to obtain a cationic solution. Approximately 0.354 g of $NaHCO_3$ was dissolved in 40 ml DDW, and 20.391 g $H_3PO_4$ was dissolved in 580 ml DDW while being stirred. After that, prepared $NaHCO_3$ solution and $H_3PO_4$ solution were added in a dropwise manner into the cationic solution in a period of approximately 270 minutes in order to obtain a first suspension. The rate of addition of $NaHCO_3$ and $H_3PO_4$ were 0.15 and 2.2 ml/min, respectively. Then the first suspension was aged for 48 hr.

After that, the first suspension was washed three times with DDW and centrifuged at 4000 rpm for approximately 20 minutes to obtain a paste. The paste was dried in an oven at a temperature of approximately 70°C, overnight to obtain a solid composition. The solid composition was then milled to obtain the Zn-Na-CHA nanopowder.

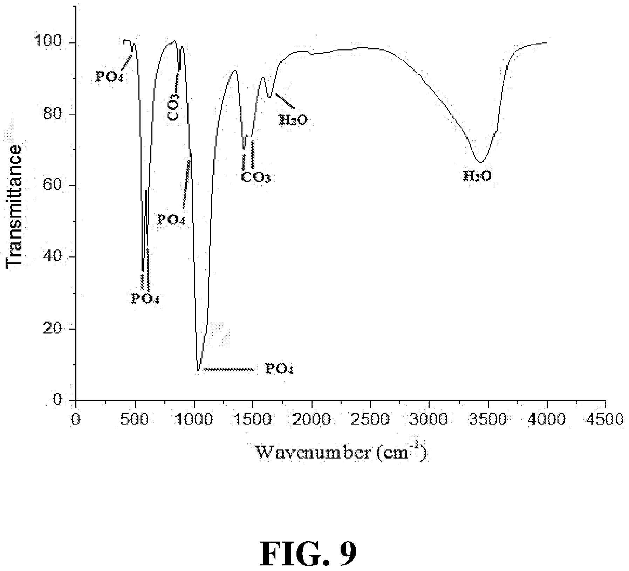

**FIG. 9** shows FTIR spectrum of the synthesized Zn-Na-CHA nanopowder. The FTIR spectrum of the synthesized Zn-Na-CHA nanopowder consist of several peaks at 471.68, 584.19, 603.54, 963.18, and 1034.62 cm$^{-1}$ which are assigned to the $PO_4^{3-}$ bands. Moreover, another peak associated with OH$^-$ functional group at 3569 cm$^{-1}$ appeared in the FTIR spectrum of the synthesized Zn-Na-CHA nanopowder. In addition, several broad peaks in the range of 3000-3500 cm$^{-1}$ and a sharp peak at 1636.16 cm$^{-1}$ could be attributed to the water which is adsorbed due to the high specific area of the synthesized Zn-Na-CHA nanopowder. Referring to **FIG. 9,** the peaks associated with $CO_3^{2-}$ functional groups appeared at 874.9, and 1421.11 cm$^{-1}$, which can be substituted to both $PO_4^{3-}$ and OH$^-$ ions.

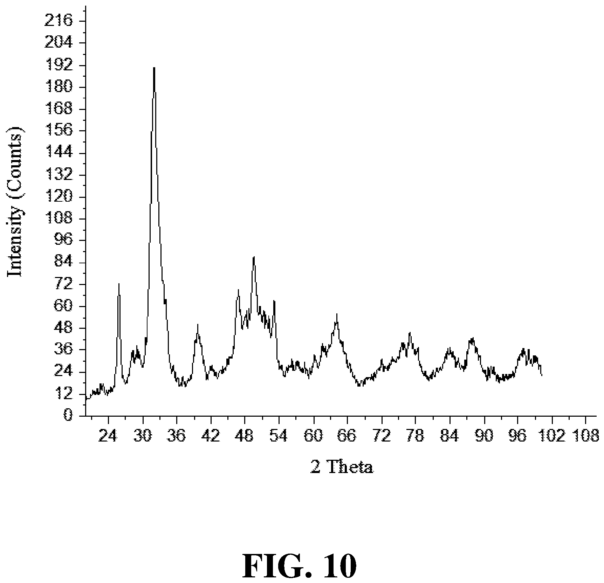

**FIG. 10** shows XRD pattern of the synthesized Zn-Na-CHA nanopowder, where all peaks were assigned to the standard pattern (JCPDS No. 09-0432). No peaks corresponding to the second phases like calcium hydroxide or zinc phosphate were detected. With reference to **FIG. 10,** the XRD data extracted from the XRD pattern of the synthesized Zn-Na-CHA nanopowder were used to calculate the lattice parameters (*a* and *c*) of the unit cells of the hexagonal crystalline structure of the synthesized Zn-Na-CHA nanopowder by Equation (1). The calculated average lattice parameters of the synthesized Zn-Na-CHA nanopowder are presented in **Table 7.**

**Table 8** reports the average lattice parameters of a stoichiometry HA, the synthesized HA in Example 1, the synthesized Mg-Na-CHA in Example 2, the synthesized Zn-Na-CHA in Example 3, along with the average lattice parameters of natural HA in enamel, dentine, and bone.

**Table 7:** Lattice parameters of the synthesized Zn-Na-CHA nanopowder.

| 2θ | h | k | l | d (Å) (from graph) | a (Å) | c (Å) |
|---|---|---|---|---|---|---|
| 18.7850 | 1 | 1 | 0 | 4.7362 | 9.4724 | 0 |
| 21.8190 | 2 | 0 | 0 | 4.0735 | 9.4073 | 0 |
| 25.8790 | 0 | 0 | 2 | 3.4372 | 0.0000 | 6.8744 |
| 28.9660 | 2 | 1 | 0 | 3.0869 | 9.4305 | 0 |
| 32.9020 | 3 | 0 | 0 | 2.7217 | 9.4284 | 0 |
| 39.8180 | 3 | 1 | 0 | 2.2718 | 9.4581 | 0 |
| 44.3690 | 4 | 0 | 0 | 2.0448 | 9.4443 | 0 |
| 48.6230 | 3 | 2 | 0 | 1.8754 | 9.4395 | 0 |
| 51.2830 | 4 | 1 | 0 | 1.7827 | 9.4332 | 0 |
| 53.1430 | 0 | 0 | 4 | 1.7233 | 0.0000 | 6.8932 |
| 59.9380 | 4 | 2 | 0 | 1.5448 | 9.4387 | 0 |
| 63.4430 | 5 | 1 | 0 | 1.4655 | 9.4216 | 0 |
| | | | | **Average** | 9.4374 | 6.8838 |

Referring to **Table 8,** the c-axes of the synthesized Zn-Na-CHA and the synthesized Mg-Na-CHA are close to that of the enamel HA, but the a-axes of the synthesized Zn-Na-CHA and the synthesized Mg-Na-CHA are close to those of the bone and dentin HA.

**Table 8:** Lattice parameters of a stoichiometry HA, the synthesized HA, the synthesized Mg-Na-CHA, the synthesized Zn-Na-CHA, the enamel HA, the dentin HA, and the bone HA.

| Sample | a (Å) | c (Å) |
|---|---|---|
| Sto-HA | 9.4185 | 6.884 |
| HA Synthetic | 9.4170 | 6.8828 |
| Mg-Na-CHA | 9.4187 | 6.87 |
| Zn-Na-CHA | 9.4172 | 6.8764 |
| Enamel HA | 9.441 | 6.880 |
| Dentin HA | 9.421 | 6.887 |
| Bone HA | 9.41 | 6.89 |

Referring to **FIGs. 8** and **10,** the XRD data were further used to calculate the degree of crystallinity of the synthesized Mg-Na-CHA and the synthesized Zn-Na-CHA nanopowders by Equation (2). The results of calculations are presented in **Table 9.** Referring to data reported in **Table 9,** it can be understood that incorporation of Zn and Mg ions in CHA crystal structure results in broadening of the diffraction pattern peaks and consequently deceases the degree of crystallinity of the synthesized Zn-Na-CHA and Mg-Na-CHA nanopowders. Peak broadening indicates a low degree of crystallinity about 46%, 27%, and 28% for the synthesized HA in Example 1, Mg-Na-CHA and Zn-Na-CHA nanopowders, respectively. While the degree of crystallinity for natural enamel HA is between 70-75%; for dentin HA is between 33-37%; and for bone HA is between 33-37%. Referring to **Table 9,** the crystallinities of the synthesized Mg-Na-CHA and Zn-Na-CHA nanopowders are close to those of dentine and bone HA

Table 9: Degree of crystallinity of synthesized HA, Zn-Na-CHA, Mg-Na-CHA, enamel HA, dentin HA, and bone HA.

| Type of powder | 2θ | variable | Intensity | Crystallinity (%) |
|---|---|---|---|---|
| HA | 32.78 | V112/300 | 56 | 46.15 |
| | 32.92 | 1300 | 104 | |
| Mg-Na-CHA | 32.9 | V112/300 | 66 | 26.67 |
| | 32.92 | 1300 | 90 | |
| Zn-Na-CHA | 32.78 | V112/300 | 85 | 28.57 |
| | 32.9 | 1300 | 119 | |
| Enamel HA | | | | 70-75 |
| Dentin HA | | | | 33-37 |
| Bone HA | | | | 33-37 |

In addition, Scherrer's equation presented as Equation (3), was used to determine the crystallite size of the synthesized HA, the synthesized Zn-Na-CHA nanopowder, the synthesized Mg-Na-CHA nanopowder, and enamel HA sample. In Equation (3), $\lambda$ is the wavelength (Cu K$\alpha$), $\beta_{1/2}$ is the full width at half-maximum of the selected peak in the XRD patterns (shown in **FIGs. 1, 8** and **10**) and $\theta$ is the diffraction angle (referred to as "theta" in **FIGs. 1, 8** and **10**). The (002), (310), and (222) peaks were used for the calculations. Results of crystallite size calculations are presented in **Table 10.** The average crystallite sizes of the synthesized Zn-Na-CHA nanopowder and the synthesized Mg-Na-CHA nanopowder are closer to that of the enamel HA.

Table 10: Crystallite size synthesized HA, Zn-Na-CHA, Mg-Na-CHA, and enamel HA

| Sample | Crystallite Size (nm) | | | |
|---|---|---|---|---|
| | 002 | 310 | 222 | Average |
| HA | 32.37 | 54.27 | 23.92 | 36.85 |
| Mg-Na-CHA | 33.36 | 25.71 | 23.52 | 27.53 |
| Zn-Na-CHA | 22.03 | 32.49 | 31.007 | 28.509 |
| Enamel HA | 21.47 | 34.91 | 26.18 | 27.52 |

**Claims**

1. A biomimetic apatite nanopowder composition, comprising hydroxyapatite particles with a formula:

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}$$

with r being between 8 and 10, m being between 0.083 and 0.25, n being between 0.17 and 0.35, z being between 0.03 and 0.05, p being between 4 and 6 such that $p - \frac{2}{3}a - s$ being between 5.66 and 5.93, a being between 0.105 and 0.52, b being between 0.17 and 0.48, s being between 0 and 0.5, wherein a and b are selected such that a+b is between 0.58 and 0.68.

2. The composition of claim 1, wherein Ca to P ratio is between 1.52:1 and 1.85:1.

3. The composition of claim 1, wherein the hydroxyapatite particles include rod-shaped particles with a diameter between 3 and 30 nm, a length between 20 and 130 nm, and a length to diameter ratio between 4:1 and 15:1.

4. The composition of claim 3, wherein Ca to P ratio is between 1.52:1 and 1.85:1.

5. A biomimetic apatite nanopowder composition, comprising hydroxyapatite particles with a formula:

$$\mathrm{Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}}$$

with r being between 8 and 10, m being between 0.4 and 0.55, n being between 0.044 and 0.26, z being between 0.015 and 0.03, p being between 4 and 6 such that p-2/3 a-s being between 5.47 and 6, a being between 0 and 0.8, b being between 0.29 and 0.9, s being between 0 and 0.5,
wherein a and b are selected such that a+b is between 0.9 and 1.09.

6. The composition of claim 5, wherein Ca to P ratio is between 1.52:1 and 1.85:1.

7. The composition of claim 5, wherein the hydroxyapatite particles include rod-shaped particles with a diameter between 3 and 30 nm, a length between 20 and 130 nm, and a length to diameter ratio between 4:1 and 15:1.

8. The composition of claim 7, wherein Ca to P ratio is between 1.52:1 and 1.85:1.

9. The composition of claim 5, wherein:

r being between 8 and 10, m being between 0.2 and 0.34, n being between 0.3 and 0.44, z being between 0.0006 and 0.006, p being between 4 and 6 such that p-2/3 a-s being between 5.17 and 5.54, a being between 0.69 and 1.24, b being between 0 and 0.4, s being between 0 and 0.018,
wherein a and b are selected such that a+b is between 1.09 and 1.24.

10. The composition of claim 8, wherein Ca to P ratio is between 1.52:1 and 1.85:1.

11. The composition of claim 9, wherein the hydroxyapatite particles include rod-shaped particles with a diameter between 3 and 30 nm, a length between 20 and 130 nm, and a length to diameter ratio between 4:1 and 15:1.

**Patentansprüche**

1. Biomimetische Apatit-Nanopulver-Zusammensetzung, umfassend Hydroxyapatit-Partikel mit einer Formel:

$$\mathrm{Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}}$$

wobei r ist zwischen 8 und 10, m ist zwischen 0.083 und 0.25, n ist zwischen 0.17 und 0.35, z ist zwischen 0.03 und 0.05, p ist zwischen 4 und 6 so daß $p - \frac{2}{3}a - s$ ist zwischen 5.66 und 5.93, a ist zwischen 0.105 und 0.52, b ist zwischen 0.17 und 0.48, s ist zwischen 0 und 0.5,
wobei a und b so ausgewählt sind, dass a+b zwischen 0.58 und 0.68 liegt.

2. Zusammensetzung nach Anspruch 1, wobei das Verhältnis von Ca zu P zwischen 1.52:1 und 1.85:1 liegt.

3. Zusammensetzung nach Anspruch 1, wobei die Hydroxyapatit-Partikel stäbchenförmige Partikel mit einem Durchmesser zwischen 3 und 30 nm, einer Länge zwischen 20 und 130 nm, und einem Verhältnis von Länge zu Durchmesser zwischen 4:1 und 15:1 umfassen.

4. Zusammensetzung nach Anspruch 3, wobei das Verhältnis von Ca zu P zwischen 1.52:1 und 1.85:1 liegt.

5. Biomimetische Apatit-Nanopulver-Zusammensetzung, umfassend Hydroxyapatit-Partikel mit einer Formel:

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}$$

mit r ist zwischen 8 und 10, m ist zwischen 0.4 und 0.55, n ist zwischen 0.044 und 0.26 , z ist zwischen 0.015 und 0.03, p ist zwischen 4 und 6, so daß $p - \frac{2}{3}a - s$ ist zwischen 5.47 und 6, a ist zwischen 0 und 0.8, b ist zwischen 0.29 und 0.9, s ist zwischen 0 und 0.5,
wobei a und b so ausgewählt sind, dass a+b zwischen 0.9 und 1.09 liegt.

**6.** Zusammensetzung nach Anspruch 5, wobei das Verhältnis von Ca zu P zwischen 1.52:1 und 1.85:1 liegt.

**7.** Zusammensetzung nach Anspruch 5, wobei die Hydroxyapatit-Partikel stäbchenförmige Partikel mit einem Durchmesser zwischen 3 und 30 nm, einer Länge zwischen 20 und 130 nm, und einem Länge-zu-Durchmesser-Verhältnis zwischen 4:1 und 15:1 umfassen.

**8.** Zusammensetzung nach Anspruch 7, wobei das Verhältnis von Ca zu P zwischen 1.52:1 und 1.85:1 liegt.

**9.** Zusammensetzung nach Anspruch 5, wobei:

r ist zwischen 8 und 10, m ist zwischen 0.2 und 0.34, n ist zwischen 0.3 und 0.44, z ist zwischen 0,0006 und 0.006 , p ist zwischen 4 und 6, so dass $p - \frac{2}{3}a - s$ ist zwischen 5.17 und 5.54, a ist zwischen 0.69 und 1.24, b ist zwischen 0 und 0.4, s ist zwischen 0 und 0.018,
wobei a und b so ausgewählt sind, dass a+b zwischen 1.09 und 1.24 liegt.

**10.** Zusammensetzung nach Anspruch 8, wobei das Verhältnis von Ca zu P zwischen 1.52:1 und 1.85:1 liegt.

**11.** Zusammensetzung nach Anspruch 9, wobei die Hydroxyapatit-Partikel stäbchenförmige Partikel mit einem Durchmesser zwischen 3 und 30 nm, einer Länge zwischen 20 und 130 nm, und einem Länge-zu-Durchmesser-Verhältnis zwischen 4:1 und 15:1 umfassen.

**Revendications**

**1.** Composition de nanopoudre d'apatite biomimétique, comprenant des particules d'hydroxyapatite de formule:

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}$$

avec r étant compris entre 8 et 10, m étant compris entre 0.083 et 0.25, n étant compris entre 0.17 et 0.35, z étant compris entre 0.03 et 0.05, p étant compris entre 4 et 6 tel qu'étant $p - \frac{2}{3}a - s$ compris entre 5.66 et 5.93, a étant entre 0.105 et 0.52, b étant entre 0.17 et 0.48, s étant compris entre 0 et 0.5,
dans laquelle a et b sont choisis de telle sorte que a+b soit compris entre 0.58 et 0.68.

**2.** Composition selon la revendication 1, dans laquelle le rapport Ca à P est compris entre 1.52:1 et 1.85:1.

**3.** Composition selon la revendication 1, dans laquelle les particules d'hydroxyapatite comprennent des particules en forme de bâtonnets d'un diamètre compris entre 3 et 30 nm, d'une longueur comprise entre 20 et 130 nm, et d'un rapport longueur/diamètre compris entre 4:1 et 15:1.

**4.** Composition selon la revendication 3, dans laquelle le rapport Ca sur P est compris entre 1.52:1 et 1.85:1.

**5.** Composition de nanopoudre d'apatite biomimétique, comprenant des particules d'hydroxyapatite de formule:

$$Ca_{r-z-m-\frac{n}{2}}Mg_mNa_nZn_z(PO_4)_{p-\frac{2}{3}a-s}(SiO_4)_s(CO_3)_{a+b}(OH)_{2-2b-s}$$

avec r étant compris entre 8 et 10, m étant entre 0.4 et 0.55, n étant compris entre 0.044 et 0.26, z étant compris entre 0.015 et 0.03, p étant compris entre 4 et 6 de telle sorte que $p-\frac{2}{3}a-s$ comme étant entre 5.47 et 6, a étant compris entre 0 et 0.8, b étant compris entre 0.29 et 0.9, s étant compris entre 0 et 0.5, dans laquelle a et b sont choisis de telle sorte que a+b soit compris entre 0.9 et 1.09.

6. Composition selon la revendication 5, dans laquelle le rapport Ca sur P est compris entre 1.52:1 et 1.85:1.

7. Composition selon la revendication 5, dans laquelle les particules d'hydroxyapatite comprennent des particules en forme de bâtonnet d'un diamètre compris entre 3 et 30 nm, d'une longueur comprise entre 20 et 130 nm, et d'un rapport longueur sur diamètre compris entre 4:1 et 15:1.

8. Composition selon la revendication 7, dans laquelle le rapport Ca à P est compris entre 1.52:1 et 1.85:1.

9. Composition selon la revendication 5, dans laquelle:

r étant compris entre 8 et 10, m étant entre 0.2 et 0.34, n étant compris entre 0.3 et 0.44, z étant compris entre 0.0006 et 0.006, p étant compris entre 4 et 6 de telle sorte que $p-\frac{2}{3}a-s$ comme étant compris entre 5.17 et 5.54, a étant compris entre 0.69 et 1.24, b étant compris entre 0 et 0.4, s étant compris entre 0 et 0.018, dans laquelle a et b sont choisis de telle sorte que a+b soit compris entre 1.09 et 1.24.

10. Composition selon la revendication 8, dans laquelle le rapport Ca sur P est compris entre 1.52:1 et 1.85:1.

11. Composition selon la revendication 9, dans laquelle les particules d'hydroxyapatite comprennent des particules en forme de bâtonnets d'un diamètre compris entre 3 et 30 nm, d'une longueur comprise entre 20 et 130 nm, et d'un rapport longueur sur diamètre compris entre 4:1 et 15:1.

FIG. 1

FIG. 2

500 nm

## FIG. 3A

**FIG. 3B**

**FIG. 4**

**FIG. 5A**

**FIG. 5B**

**FIG. 5C**

**FIG. 5D**

**FIG. 5E**

FIG. 6

**FIG. 7**

**FIG. 8**

**FIG. 9**

**FIG. 10**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHEPHERD et al.** *Biomedical Materials,* 2013, vol. 8.2, 025003
- **FRIEDERICHS et al.** *Journal of The Royal Society Interface,* 2015, vol. 12.108, 20150190
- **FADEEV et al.** *Inorganic Materials,* 2003, vol. 39.9, 947-950
- **SPIRO et al.** *Materials Science and Engineering,* 2008, 179-187
- **ZYMAN et al.** *Processing and Application of Ceramics,* 2013, vol. 7.4, 153-157
- **CHEN et al.** *Nanoscale research letters,* 2015, vol. 10 (1), 1-6